# EUROPEAN PATENT APPLICATION

(11) **EP 4 840 944 A1**
(43) Date of publication of application: **18.11.2026**
(21) Application number: 25738321.6
(22) Date of filing: 03.01.2025
(51) Int. Cl.: C12N 5/10, C12N 5/0783, C12N 15/62, A61K 35/17, A61P 35/00

(54) **ENGINEERED TIL CELL HAVING IMPROVED TUMOR RECOGNITION AND KILLING CAPABILITY AND USE THEREOF**

(30) Priority: 08.01.2024 CN 202410022305
(71) Applicant: Shanghai Chuangyinsai Biotechnology Co., Ltd., Shanghai 201210 (CN)
(72) Inventor: XU, Aotian, Qingdao, Shandong 266109 (CN); YU, Zhongjie, Qingdao, Shandong 266109 (CN); ZHAO, Yi, Qingdao, Shandong 266109 (CN); GAO, Qing, Qingdao, Shandong 266109 (CN)
(74) Representative: dompatent
(86) International application number: PCT/CN2025/070361
(87) International publication number: WO 2025/148790

(57) **Abstract**

Provided are an engineered TIL cell having an improved tumor recognition and killing capability and a use thereof, relating to the technical field of genetic engineering and cell therapy. The surface of the engineered TIL cell comprises an exogenous membrane-bound DAP10-CD3ζ fusion protein, and the endoplasmic reticulum or Golgi apparatus of the engineered TIL cell comprises a retained fusion protein. Specifically, provided is the membrane-bound DAP10-CD3ζ fusion protein, so that the TIL cell can be activated by an activated fusion protein when recognizing a tumor cell by means of the NKG2D-NKG2DL pathway, so as to effectively kill the tumor cell. Further provided is the retained fusion protein, so that endogenous NKG2DL that is expressed when the TIL cell is activated can bind to the retained fusion protein so as to be retained in the cell, thereby avoiding mutual recognition and killing between the TIL cells and improving the viability of the TIL cells.

## Description

### Technical Field

The present invention relates to the technical fields of genetic engineering and cell therapy, and specifically to an engineered TIL cell having improved tumor recognition and killing capability and use thereof.

### Background

Tumor-infiltrating lymphocyte (TIL) therapy refers to a therapy where lymphocytes infiltrating a tumor tissue are isolated, cultured and expanded *in vitro*, and then infused back into a patient. TILs are composed of T cells with diverse TCR clones, which can recognize multiple tumor-specific neoantigens and tumor-associated antigens, thereby enabling them to more effectively address tumor heterogeneity. TILs usually comprise a certain proportion of effector memory T cells. Upon stimulation by tumor antigens *in vivo*, they express chemokine receptors, which facilitates their homing to the tumor tissue after infusion. Furthermore, since TILs are derived from the patient's own body, they have lower toxicity. TIL therapy has shown great potential in the field of solid tumors.

TIL cells derived from tumor tissues are rich in tumor-specific T cells. However, these tumor-specific T cells often become terminally differentiated and exhausted after tumor-specific activation, leading to a reduced killing ability of the TIL cells against tumor cells. Furthermore, a large proportion of cells in the cultured TIL cells are non-tumor-specific bystander cells. How to convert bystander cells into effector cells capable of recognizing and killing tumor cells is an urgent problem to be solved. Additionally, tumor cells often downregulate MHC-I molecule expression or harbor TAP gene mutations, leading to loss of antigen presentation function, thereby evading immune cell-mediated killing.

The NKG2D molecules on the surface of TIL cells can bind to the NKG2DL molecules on the surface of tumor cells, thereby enabling the TIL cells to recognize tumor cells. However, TIL cells transiently express NKG2DL when activated, which causes fratricide between TIL cells, leading to a significant decrease in TIL cell viability.

Therefore, there is an urgent need in the art to develop engineered TIL cells that have the function of recognizing and killing tumor cells, reduce immune evasion, and can reduce the fratricide of TIL cells.

### Summary of the Invention

The purpose of the present invention is to provide engineered TIL cells that have the function of recognizing and killing tumor cells, reduce immune evasion, and can reduce the fratricide of TIL cells, as well as use thereof.

In a first aspect of the present invention, an engineered tumor-infiltrating lymphocyte (TIL cell) is provided, wherein the engineered TIL cell expresses an exogenous fusion protein selected from the group consisting of: an activation fusion protein, a retention fusion protein, and a combination thereof;
wherein the activation fusion protein is a membrane-bound DAP10-CD3ζ fusion protein;
and wherein the retention fusion protein comprises: an NKG2D extracellular domain and a KDEL sequence.

In another preferred embodiment, the engineered TIL cell expresses both the activation fusion protein and the retention fusion protein.

In another preferred embodiment, the activation fusion protein comprises: (1) an extracellular domain, wherein the extracellular domain comprises a DAP10 element or an active fragment thereof;
(2) a transmembrane domain; and
(3) an intracellular signaling domain, wherein the intracellular signaling domain comprises a DAP10 intracellular domain and CD3ζ;
wherein the extracellular domain and the intracellular signaling domain are linked in tandem via the transmembrane domain.

In another preferred embodiment, the engineered TIL cell has the activation fusion protein on its cell membrane; and/or the engineered TIL cell has the retention fusion protein in its endoplasmic reticulum or Golgi apparatus.

In another preferred embodiment, the engineered TIL cell comprises a polynucleotide encoding the activation fusion protein and/or the retention fusion protein.

In another preferred embodiment, the engineered TIL cell comprises a nucleic acid construct, wherein the nucleic acid construct comprises a first expression cassette comprising a polynucleotide encoding the activation fusion protein and/or a polynucleotide encoding the retention fusion protein.

In another preferred embodiment, the engineered TIL cell comprises a vector comprising: a polynucleotide encoding the activation fusion protein and/or a polynucleotide encoding the retention fusion protein.

In another preferred embodiment, the engineered TIL cell further expresses a membrane-bound IL-15 fusion protein comprising the following elements:
(i) interleukin 15 or a functionally active fragment thereof;
(ii) a transmembrane domain; and
(iii) an intracellular domain;
wherein the intracellular domain is a CD86 intracellular domain.

In another preferred embodiment, the CD86 intracellular domain comprises the full-length intracellular domain or an active fragment thereof.

In another preferred embodiment, IL-15 comprises full-length, mature form of IL-15, or an active fragment thereof.

In another preferred embodiment, IL-15 comprises wild-type and mutant forms.

In another preferred embodiment, the amino acid sequence of IL-15 is set forth in SEQ ID NO: 6.

In another preferred embodiment, the fusion polypeptide further comprises a hinge region.

In another preferred embodiment, the fusion polypeptide further optionally comprises a signal peptide and/or a linker.

In another preferred embodiment, the linker is located between elements (i) and (ii), between elements (ii) and (iii), between element (i) and the hinge region, or between the hinge region and element (ii).

In another preferred embodiment, the linker comprises a flexible linker or a rigid linker.

In another preferred embodiment, the amino acid sequence of the linker is set forth in SEQ ID NO: 7.

In another preferred embodiment, the structure of the fusion polypeptide is shown in Formula (I):

X-IL15-L-H-TM-Cyto (I)

wherein,
"-" is each independently absent or a linking peptide;
X is absent or a signal peptide;
IL15 is an interleukin 15 element;
L is absent or a linker;
H is a hinge region;
TM is a transmembrane domain;
Cyto is an intracellular domain, wherein the intracellular domain is a CD86 intracellular domain.

In another preferred embodiment, the transmembrane domain is a CD86 transmembrane domain.

In another preferred embodiment, the amino acid sequence of the CD86 transmembrane domain and intracellular domain is set forth in SEQ ID NO: 9.

In another preferred embodiment, the hinge region is a CD86 hinge region, preferably, the amino acid sequence of the CD86 hinge region is set forth in SEQ ID NO: 7.

In another preferred embodiment, the amino acid sequence of the signal peptide is set forth in SEQ ID NO: 5.

In another preferred embodiment, the engineered TIL cell has one or more properties selected from the group consisting of:
(1) enhanced ability to recognize and kill tumor cells;
(2) reduced ability of activated TIL cells to recognize and kill each other;
(3) broadened target antigen recognition spectrum of TIL cells;
(4) effective prevention of tumor immune evasion and recurrence.

In a second aspect of the present invention, an activation fusion protein is provided, wherein the activation fusion protein comprises:
(1) an extracellular domain, wherein the extracellular domain comprises a DAP10 element or an active fragment thereof;
(2) a transmembrane domain; and
(3) an intracellular signaling domain, wherein the intracellular signaling domain comprises a DAP10 intracellular domain and CD3ζ;
wherein the extracellular domain and the intracellular signaling domain are linked in tandem via the transmembrane domain.

In another preferred embodiment, in the intracellular signaling domain, the DAP10 intracellular domain and CD3ζ are linked directly or linked via a linking peptide. (Preferably, the length of the linking peptide is 1-10 aa, more preferably 1-6 aa, even more preferably 1-3 aa).

In another preferred embodiment, the intracellular signaling domain further comprises a co-stimulatory domain.

In another preferred embodiment, the intracellular signaling domain further comprises one, two, or more co-stimulatory domains.

In another preferred embodiment, the co-stimulatory domain is a co-stimulatory domain from a protein selected from the group consisting of: CD28, CD27, 4-1BB, CD40, OX40, and combinations thereof.

In another preferred embodiment, the co-stimulatory domain is located between the DAP10 intracellular domain and CD3ζ, and/or at the C-terminus of CD3ζ.

In another preferred embodiment, when the intracellular signaling domain comprises an additional co-stimulatory domain, the DAP10 intracellular domain and the co-stimulatory domain, and/or CD3ζ and the co-stimulatory domain, may be linked directly or linked via a linking peptide; preferably, the length of the linking peptide is 1-10 aa, more preferably 1-6 aa, most preferably 1-3 aa.

In another preferred embodiment, the fusion protein further comprises a signal peptide at the N-terminus.

In another preferred embodiment, the signal peptide is selected from the group consisting of: DAP10 signal peptide, CD8a signal peptide, and CD28 signal peptide.

In another preferred embodiment, the amino acid sequence of the signal peptide is set forth in positions 1-18 of SEQ ID NO: 1.

In another preferred embodiment, the amino acid sequence of the extracellular domain is set forth in positions 19-48 of SEQ ID NO: 1.

In another preferred embodiment, the transmembrane domain is a transmembrane domain from DAP10, CD8a, or CD28.

In another preferred embodiment, the amino acid sequence of the transmembrane domain is set forth in positions 49-69 of SEQ ID NO: 1.

In another preferred embodiment, the amino acid sequence of the DAP10 intracellular domain in the intracellular signaling domain is set forth in positions 70-92 of SEQ ID NO: 1.

In another preferred embodiment, the amino acid sequence of CD3ζ in the intracellular signaling domain is set forth in positions 93-206 of SEQ ID NO: 1.

In another preferred embodiment, the amino acid sequence of the intracellular signaling domain is set forth in positions 70-206 of SEQ ID NO: 1.

In another preferred embodiment, the amino acid sequence of the fusion polypeptide is set forth in SEQ ID NO: 1.

In another preferred embodiment, the fusion polypeptide has one or more properties selected from the group consisting of:
(a) the fusion protein has the function of binding NKG2D;
(b) activating T cells (TIL cells);
(c) activating NK cells.

In another preferred embodiment, the activation is self-activation (i.e., the immune cell expressing the membrane-bound DAP10-CD3ζ fusion protein is activated).

In another preferred embodiment, the activated immune cell (T cell, NK cell) can effectively kill target cells through the interaction of NKG2D-NKG2DLs.

In a third aspect of the present invention, a retention fusion protein is provided, wherein the retention fusion protein comprises: an NKG2D extracellular domain and a KDEL sequence.

In another preferred embodiment, the KDEL sequence in the retention fusion protein is located at the C-terminus of the NKG2D extracellular domain.

In another preferred embodiment, in the retention fusion protein, the NKG2D extracellular domain is set forth in positions 22-156 of SEQ ID NO: 2.

In another preferred embodiment, the NKG2D extracellular domain and the KDEL sequence are linked directly or linked via a linking peptide; preferably, the length of the linking peptide is 1-15 aa, more preferably 1-10 aa, most preferably 1-5 aa.

In another preferred embodiment, the KDEL sequence is located at positions 157-160 of SEQ ID NO: 2.

In another preferred embodiment, the amino acid sequence of the retention fusion protein is set forth in SEQ ID NO: 2.

In another preferred embodiment, the retention fusion protein further comprises a signal peptide; preferably a CD8a signal peptide; more preferably the signal peptide is as set forth in SEQ ID NO: 3.

In another preferred embodiment, the retention fusion protein can bind to endogenous NKG2DL, thereby retaining the endogenous NKG2DL intracellularly.

In a fourth aspect of the present invention, a polynucleotide is provided, wherein the polynucleotide encodes the activation fusion protein according to the second aspect of the present invention and/or the retention fusion protein according to the third aspect of the present invention.

In another preferred embodiment, the polynucleotide encodes the activation fusion protein according to the second aspect of the present invention.

In another preferred embodiment, the polynucleotide encodes the retention fusion protein according to the third aspect of the present invention.

In another preferred embodiment, the polynucleotide encodes the activation fusion protein according to the second aspect of the present invention and the retention fusion protein according to the third aspect of the present invention.

In another preferred embodiment, the activation fusion protein and the retention fusion protein are linked via a cleavable linking peptide.

In another preferred embodiment, the cleavable linking peptide is a self-cleaving peptide; a preferred self-cleaving peptide is selected from the group consisting of: T2A peptide, P2A peptide, and a combination thereof.

In a fifth aspect of the present invention, a nucleic acid construct is provided, wherein the nucleic acid construct comprises a first expression cassette comprising the polynucleotide according to the fourth aspect of the present invention.

In another preferred embodiment, the first expression cassette further comprises a promoter.

In another preferred embodiment, the promoter is operably linked to the polynucleotide.

In another preferred embodiment, the promoter is selected from the group consisting of: a constitutive promoter, an inducible promoter, and a combination thereof.

In another preferred embodiment, the promoter comprises a hypoxia-responsive promoter.

In another preferred embodiment, the hypoxia-responsive promoter is a promoter comprising n hypoxia response elements (HREs), i.e., a promoter containing n×HRE elements, where n is an integer selected from 1 to 20.

In another preferred embodiment, n is an integer selected from 2 to 9; more preferably, n is an integer selected from 2 to 4; most preferably, n is 3.

In another preferred embodiment, the hypoxia-responsive promoter is selected from the group consisting of: a TK-mini promoter containing n×HRE elements, a CMV-mini promoter containing n×HRE elements, and an IL2-mini promoter containing n×HRE elements.

In another preferred embodiment, the hypoxia-responsive promoter is a 3×HRE TK-mini promoter.

In another preferred embodiment, the nucleic acid construct further comprises a molecular switch element sequence selected from the group consisting of: hEGFRt, BCMA, and CD20.

In another preferred embodiment, the molecular switch element is hEGFRt.

In another preferred embodiment, the molecular switch element is linked to the first expression cassette via a cleavable linking peptide sequence.

In another preferred embodiment, the cleavable linking peptide is a self-cleaving 2A peptide; preferably a T2A peptide.

In another preferred embodiment, the nucleic acid construct comprises a second expression cassette comprising a second promoter and a coding sequence for the molecular switch element.

In another preferred embodiment, the second promoter is an SFFV promoter.

In a sixth aspect of the present invention, a vector is provided, wherein the vector comprises the polynucleotide according to the fourth aspect of the present invention.

In another preferred embodiment, the vector comprises a plasmid and a viral vector.

In another preferred embodiment, the viral vector comprises: a lentiviral vector, an adenoviral vector, and a yellow fever viral vector.

In another preferred embodiment, the vector is a plasmid.

In a seventh aspect of the present invention, a composition is provided, wherein the composition comprises the engineered TIL cell according to the first aspect of the present invention, the activation fusion protein according to the second aspect of the present invention, the retention fusion protein according to the third aspect of the present invention, the polynucleotide according to the fourth aspect of the present invention, the nucleic acid construct according to the fifth aspect of the present invention, and/or the vector according to the sixth aspect of the present invention, and a pharmaceutically acceptable carrier.

In another preferred embodiment, the composition comprises the engineered TIL cell according to the first aspect of the present invention and a pharmaceutically acceptable carrier.

In an eighth aspect of the present invention, a kit is provided, wherein the kit comprises the engineered TIL cell according to the first aspect of the present invention, the activation fusion protein according to the second aspect of the present invention, the retention fusion protein according to the third aspect of the present invention, the polynucleotide according to the fourth aspect of the present invention, the nucleic acid construct according to the fifth aspect of the present invention, the vector according to the sixth aspect of the present invention, and/or the composition according to the seventh aspect of the present invention.

In another preferred embodiment, the kit comprises the engineered TIL cell according to the first aspect of the present invention, or a reagent for preparing the engineered TIL cell according to the first aspect of the present invention, wherein the reagent is selected from the group consisting of:
(Y1) a polynucleotide encoding the activation fusion protein and the retention fusion protein; or
(Y2) a vector comprising: a polynucleotide encoding the activation fusion protein and/or a polynucleotide encoding the retention fusion protein.

In a ninth aspect of the present invention, use of the engineered TIL cell according to the first aspect of the present invention, the activation fusion protein according to the second aspect of the present invention, the retention fusion protein according to the third aspect of the present invention, the polynucleotide according to the fourth aspect of the present invention, the nucleic acid construct according to the fifth aspect of the present invention, the vector according to the sixth aspect of the present invention, the composition according to the seventh aspect of the present invention, and/or the kit according to the eighth aspect of the present invention in the manufacture of a medicament for preventing, alleviating, and/or treating a tumor is provided.

In another preferred embodiment, use of the engineered TIL cell according to the present invention, or the kit according to the present invention in the manufacture of a medicament for treating a tumor is provided.

In another preferred embodiment, the tumor is selected from the group consisting of: lung cancer, cervical cancer, gastrointestinal cancer, pancreatic cancer, glioblastoma, ovarian cancer, bladder cancer, liver tumor, breast cancer, colon cancer, rectal cancer, colorectal cancer, endometrial or uterine cancer, salivary gland cancer, kidney cancer, prostate cancer, vulvar cancer, thyroid cancer, liver cancer, anal cancer, penile cancer, testicular cancer, esophageal cancer, bile duct tumor, and head and neck cancer.

In a tenth aspect of the present invention, a method of treating a disease is provided, wherein the method comprises administering to a subject in need thereof the cell according to the first aspect of the present invention and/or the composition according to the seventh aspect of the present invention.

In another preferred embodiment, the subject is a human or a mammal.

In another preferred embodiment, the disease is a tumor or cancer.

In another preferred embodiment, the tumor is selected from the group consisting of: lung cancer, cervical cancer, gastrointestinal cancer, pancreatic cancer, glioblastoma, ovarian cancer, bladder cancer, liver tumor, breast cancer, colon cancer, rectal cancer, colorectal cancer, endometrial or uterine cancer, salivary gland cancer, kidney cancer, prostate cancer, vulvar cancer, thyroid cancer, liver cancer, anal cancer, penile cancer, testicular cancer, esophageal cancer, bile duct tumor, and head and neck cancer.

It should be understood that within the scope of the present invention, the above-mentioned technical features of the present invention and the technical features specifically described below (e.g., in the Examples) can be combined with each other to form new or preferred technical solutions. Due to space limitations, they will not be repeated here.

### Brief Description of Drawings

Figure 1 shows a schematic diagram illustrating the engineered TILs of the present invention in killing tumor cells.
Figure 2 shows a schematic diagram illustrating the structures of constructs 055, 073, 076, and 083 of the present invention.
Figure 3 shows the detection results of the positive rate of lentivirus-infected TILs by flow cytometry, where TIL mock serves as the control TIL. The horizontal and vertical axes represent relative fluorescence intensity, and the trapezoid indicates the proportion of positive cells.
Figure 4 shows the detection results of the proliferation ability of TIL cells in each group, where TIL-MOCK serves as the control TIL.
Figure 5 shows the detection results the viability of TIL cells in each group, where TIL-MOCK serves as the control TIL.
Figure 6 shows the detection results of the proportion of stem-like cells among TIL cells in each group, where TIL-MOCK serves as the control TIL. The horizontal and vertical axes represent relative fluorescence intensity, and the Q2 region indicates the proportion of CD45RA and CD62L double-positive cells, representing the proportion of stem-like T cells.
Figure 7 shows the detection results of IFN-γ secretion by TIL cells in each group, where Ctr MOCK serves as the control TIL.
Figure 8 shows microscope observation images after co-culturing TIL cells from each group with tumor cells (HELA) at a ratio of 3:1 for 24 hours, where TIL-MOCK serves as the control TIL.
Figure 9 shows the results of cell killing assays of TIL cells from each group against tumor cells (HELA), where HELA only indicates the presence of tumor cells alone, and TIL-MOCK serves as the control TIL.
Figure 10 shows the results of cell killing assays of TIL cells from each group against tumor cells (A-375 B2M KO).
Figure 11 shows the functional effect of the molecular switch of the present invention. The horizontal and vertical axes represent relative fluorescence intensity, and the (Q2+Q3) region indicates the proportion of apoptotic cells.

### Detailed Description

After extensive and in-depth research, the present inventors have for the first time provided an engineered TIL cell, wherein the cell surface contains an exogenous membrane-bound DAP10-CD3ζ fusion protein, and its endoplasmic reticulum or Golgi apparatus contains a retention fusion protein. When expressed in TIL cells, the membrane-bound DAP10-CD3ζ fusion protein (the activation fusion protein) of the present invention can unexpectedly and effectively initiate the activation of TIL cells, thereby enabling more effective killing of tumor cells. Specifically, when the NKG2D on the surface of the engineered TIL cell of the present invention recognizes NKG2DL on the surface of a tumor cell, the TIL cell can be further effectively activated via the fusion protein of the present invention, thereby enabling the TIL cell to effectively kill the tumor cell. Experiments show that the retention fusion protein of the present invention can be effectively retained in the endoplasmic reticulum, thereby retaining the endogenously expressed NKG2DL of the TIL cell inside the cell. This avoids fratricide among TIL cells and prevents fragility during the TIL cell culture process, thus significantly improving the viability of TIL cells. The engineered TIL cells of the present invention can be used to treat tumors. Based on this, the present invention has been accomplished.

### DAP10

DAP10 is widely expressed on immune cells, is highly conserved during evolution, and exerts its unique intracellular signaling function by binding to cellular receptors such as NKG2D. DAP10 transmits cell activation signals through its YINM motif and provides co-stimulatory signals in T cells.

The amino acid sequence of human DAP10 is shown in the GenBank Accession No: AAD46986.1, consisting of 92 amino acids, as set forth in positions 1-92 of SEQ ID NO: 1. DAP10 is composed of a signal peptide (positions 1-18), an extracellular domain (positions 19-48), a transmembrane domain (positions 49-69), and an intracellular domain (positions 70-92), wherein the intracellular domain comprises a YINM motif.

The sequences of DAP10 from mammals (especially primates) are highly homologous and have similar functions. The homology between human and non-human primate DAP10 is as high as 90-100%, and the homology between human and rodent DAP10 is about 80%.

In the present invention, DAP10 comprises wild-type and mutant DAP10, provided that the mutant DAP10 retains or substantially retains the function of wild-type DAP10, e.g., retains ≥50% (preferably ≥60%, ≥70%, ≥80%, ≥90%, or even ≥100% (e.g., 100-200%)) of wild-type DAP10 function. It should be understood that in the present invention, DAP10 also comprises mutant proteins whose function exceeds that of wild-type DAP10, e.g., having ≥100% (e.g., 100-200%) of the function of wild-type DAP10.

In the present invention, mutations in DAP10 can occur naturally or be introduced artificially.

### NKG2D and NKG2DL

NKG2D is an activating receptor expressed on the surface of NK cells, NKT cells, and CD8+ T cells. It plays an important role in innate immunity and participates in the killing of tumor cells by various immune cells. The NKG2D ligand (NKG2DL) protein family mainly comprises six kinds of cytomegalovirus UL16-binding proteins 1-6 and MHC I chain-related molecules A and B (MICA, MICB). NKG2DL is barely expressed on normal cells but is highly expressed on the surface of various tumor cells of different origins (including but not limited to colorectal cancer, liver cancer, glioma, etc.).

NKG2D is a receptor associated with DAP10. The receptor complex is a hexamer consisting of an NKG2D homodimer and two DAP10 homodimers.

After NKG2D binds to NKG2DL on the surface of tumor cells, the charged amino acid residues in the transmembrane region of the NKG2D homodimer connect to the TM residues of DAP10 via two salt bridges, forming a hexameric structure, which then induces phosphorylation of the YINM motif in the cytoplasm, subsequently activating the downstream phosphoinositide 3-kinase (PI3K) signaling pathway and transmitting activation signals. The signal generated by NKG2D can directly activate NK cells to exert a killing effect, and act as a co-stimulatory signal to promote the activation of αβ T cells and γδ T cells and enhance their killing effect.

NK and NKT cells can kill tumor cells through NKG2D-NKG2DL binding. CD8+ T cells can recognize and kill tumors that do not express MHC class I molecules via NKG2D-NKG2DL.

However, TIL cells themselves highly express the NKG2D molecule, but because the downstream molecule of NKG2D, DAP10 only provides a co-stimulatory signal in T cells, TIL cells cannot effectively kill tumor cells after recognizing them via the NKG2D-NKG2DL pathway due to the lack of the first signal for T cell activation.

In the present invention, an engineered TIL cell is provided that can be activated via the NKG2D-NKG2DL pathway to kill tumor cells.

### DAP10 and CD3ζ Fusion Protein of the Present Invention

As used herein, the terms "activation fusion protein of the present invention", "membrane-bound DAP10-CD3ζ fusion protein of the present invention", and "DAP10 and CD3ζ fusion protein of the present invention" are used interchangeably and all refer to the DAP10 and CD3ζ fusion protein with T cell activation function according to the present invention. The membrane-bound DAP10-CD3ζ fusion protein of the present invention has a DAP10-derived extracellular domain, transmembrane domain, intracellular domain, and a CD3-derived intracellular structure, such as CD3ζ.

In the membrane-bound DAP10-CD3ζ fusion protein of the present invention, its transmembrane region can be the transmembrane region from DAP10, the transmembrane region from CD3, or the transmembrane region from other membrane proteins, or an artificially synthesized transmembrane region.

Furthermore, the membrane-bound DAP10-CD3ζ fusion protein of the present invention may or may not comprise a hinge region or an additional linking region between the extracellular domain and the transmembrane region, and may or may not comprise an additional linking region between the intracellular structure (e.g., CD3ζ) and the transmembrane region.

In a preferred embodiment, the extracellular domain and transmembrane region of the membrane-bound DAP10-CD3ζ fusion protein of the present invention are both from the DAP10 protein, preferably from the human DAP10 protein, such as amino acids 1-69 of the human DAP10 protein.

Typically, the activation fusion protein of the present invention comprises the following elements:
(1) a DAP10 element or an active fragment thereof; and
(2) an intracellular signaling domain derived from the DAP10 intracellular segment and CD3ζ.

In another preferred embodiment, the membrane-bound DAP10-CD3ζ fusion protein of the present invention comprises, linked in tandem from the N-terminus to the C-terminus: (1) an extracellular domain, a transmembrane domain, and an intracellular domain derived from a DAP10 element or an active fragment thereof; and (2) an intracellular signaling domain derived from CD3ζ.

In one embodiment, the intracellular signaling domain optionally further comprises a co-stimulatory molecule; the co-stimulatory molecule is selected from the group consisting of: CD28, CD27, 4-1BB, CD40, and OX40.

The amino acid sequence of a preferred activation fusion protein of the present invention is as follows: 
MIHLGHILFLLLLPVAAAQTTPGERSSLPAFYPGTSGSCSGCGSLSLPLLA
GLVAADAVASLLIVGAVFLCARPRRSPAQDGKVYINMPGRGMRVKFSRSAD
APAYQQGQNQLYNELNLGRREEYDVLDKRRGRDPEMGGKPQRRKNPQEGL
YNELQKDKMAEAYSEIGMKGERRRGKGHDGLYQGLSTATKDTYDALHMQ
ALPPR (SEQ ID NO: 1);
 wherein the signal peptide sequence is set forth in positions 1-18 of SEQ ID NO: 1, the extracellular domain sequence is set forth in positions 19-48 of SEQ ID NO: 1, the transmembrane domain sequence is set forth in positions 49-69 of SEQ ID NO: 1, the DAP10 intracellular domain sequence is set forth in positions 70-92 of SEQ ID NO: 1, and the CD3ζ sequence is set forth in positions 93-206 of SEQ ID NO: 1.

The membrane-bound DAP10-CD3ζ fusion protein of the present invention can act as a signal for TIL cell activation. Specifically, after NKG2D on the TIL cell binds to NKG2DL on the surface of a tumor cell, the TIL cell can be efficiently activated via the membrane-bound DAP10-CD3ζ fusion protein of the present invention, enabling it to kill the tumor cell.

### Retention Fusion Protein (tNKG2D-KDEL) of the Present Invention

NKG2D ligands (NKG2DLs) are not only highly expressed on the surface of tumor cells but are also transiently expressed on activated TIL cells, causing activated TIL cells to recognize and kill each other.

In the present invention, a structurally unique retention fusion protein (referred to as "the retention fusion protein of the present invention") is provided, which comprises an NKG2D extracellular domain element and a KDEL element linked in tandem.

The retention fusion protein of the present invention can retain endogenous NKG2DL produced by T cells (e.g., TIL cells) inside the cell, reducing or even eliminating NKG2DL appearing on the surface of TIL cells.

In the present invention, KDEL is Lys-Asp-Glu-Leu (also known as the KDEL signal sequence), usually located at the carboxyl terminus (C-terminus) of a protein. The KDEL sequence has a corresponding receptor on the membrane of the Golgi apparatus. Once it enters the Golgi apparatus, it binds to the receptor on the Golgi apparatus, forming a retrograde vesicle that is transported back to the endoplasmic reticulum. The KDEL sequence is referred to as an endoplasmic reticulum retention sequence.

Preferably, the present invention provides a retention fusion protein expressed inside TIL cells, wherein the retention fusion protein fuses the extracellular domain of NKG2D with the KDEL signal sequence to form a tNKG2D-KDEL protein.

After TIL cells are activated, the transiently expressed endogenous NKG2DL specifically binds to the NKG2D extracellular domain in the retention fusion protein of the present invention, thereby being retained within the endoplasmic reticulum, thus preventing or eliminating the appearance of expressed endogenous NKG2DL on the TIL cell membrane. This effectively prevents mutual recognition and fratricide among TIL cells, reduces the occurrence of cellular fragility, and thereby increases the viability of TIL cells.

The amino acid sequence of a representative retention fusion protein of the present invention is as follows: 
MALPVTALLLPLALLLHAARPLFNQEVQIPLTESYCGPCPKNWICYKNN
CYQFFDESKNWYESQASCMSQNASLLKVYSKEDQDLLKLVKSYHWMGLVH
IPTNGSWQWEDGSILSPNLLTIIEMQKGDCALYASSFKGYIENCSTPNTYICM
QRTVKDEL (SEQ ID NO: 2);
 wherein positions 22 to 156 in SEQ ID NO: 2 represent the NKG2D extracellular domain sequence; positions 157 to 160 represent the KDEL sequence.

Preferably, the retention fusion protein of the present invention further comprises a signal peptide sequence. A preferred signal peptide is the CD8a signal peptide, whose sequence is MALPVTALLLPLALLLHAARP (SEQ ID NO: 3).

### Nucleic Acid Construct of the Present Invention

As used herein, the nucleic acid construct of the present invention can express the fusion protein and/or the retention fusion protein of the present invention. The nucleic acid construct of the present invention comprises sequences encoding the fusion protein and/or the retention fusion protein of the present invention, and optionally comprises a hypoxia-responsive promoter.

The hypoxia-responsive promoter of the present invention is a promoter comprising n hypoxia-responsive elements HREs, i.e., an n×HRE promoter. Hypoxia is a hallmark feature of solid tumors, and this unique environmental signal can be used for cancer-targeted therapy, enabling the target gene to be highly expressed in the tumor microenvironment but not or lowly expressed in normal tissues, thereby maximizing the function of the exogenous gene while minimizing potential side effects. When constructing the nucleic acid molecule expressing the fusion protein of the present invention, the hypoxia-responsive promoter was used to express the fusion protein of the present invention under hypoxic conditions. There is no particular restriction on the type of promoter used to construct the hypoxia-responsive promoter of the present invention. Preferably, the present invention employs a TK mini promoter containing n×HRE.

Preferably, the present invention employs the 3×HRE-TK-mini promoter.

In one embodiment, the nucleic acid construct of the present invention further comprises a molecular switch element (also known as an immune brake element). When a safety risk arises with cells expressing the nucleic acid construct, the molecular switch element can serve as a target, and corresponding drugs can be used to eliminate the cells at risk.

In one embodiment, the molecular switch element of the present invention can be linked to the first expression cassette via a cleavable linking peptide; preferably, the cleavable linking peptide is a self-cleaving peptide; preferably, the self-cleaving peptide is selected from: T2A, P2A, or a combination thereof.

In one embodiment, the molecular switch element of the present invention is located in a second expression cassette, and expression of the molecular switch element is controlled by a second promoter. In one embodiment, the second promoter is an SFFV promoter. SFFV is a constitutive promoter that can stably and highly drive expression of the molecular switch element. In one embodiment, the second expression cassette comprises a second signal peptide.

In one embodiment, the molecular switch or immune brake element of the present invention is hEGFRt. When cells comprising the nucleic acid construct of the present invention present a safety risk, the hEGFRt therapeutic monoclonal antibody cetuximab can be injected to clear the target cells via ADCC and CDC effects, further enhancing safety. At the same time, the hEGFRt molecule can also be used for flow cytometry detection, indicating the proportion of cells positive for target gene integration.

### Membrane-Bound IL-15

In the nucleic acid construct of the present invention, preferably, it further comprises a third expression cassette expressing membrane-bound IL-15 (mIL-15), i.e., a fusion protein comprising IL-15, a transmembrane domain, and an intracellular domain. In another preferred embodiment, the membrane-bound IL-15 is also linked to the first expression cassette via a cleavable linking peptide.

Interleukin-15 (IL-15) is a pro-survival cytokine that maintains long-lived CD8+ memory T cell homeostasis, inhibits activation-induced cell death (AICD), enhances in vivo anti-tumor activity, and reverses T cell anergy. Monomeric IL-15 is a small unstable protein with a short serum half-life, requiring supraphysiological administration to achieve *in vivo* responses. In the present invention, IL-15 is linked to a transmembrane domain and an intracellular domain to obtain membrane-bound IL-15, which has the function of maintaining the long-term persistence of the memory stem cell phenotype. Preferred intracellular domain is a CD86 intracellular domain, and preferred transmembrane domain is a CD86 transmembrane domain.

The membrane-bound IL-15 can increase the proportion of stem-like T cells and reduce the expression of T cell exhaustion markers.

The membrane-bound IL-15 may further comprise a hinge region, a signal peptide, and/or a linker. Preferably, the structure of the membrane-bound IL-15 is as shown in Formula (I):

X-IL-15-L-H-TM-Cyto (I)

wherein,
"-" is each independently absent or a linking peptide;
X is absent or a signal peptide;
IL-15 is interleukin 15;
L is absent or a linker;
H is a hinge region; a preferred hinge region is the CD86 hinge region;
TM is a transmembrane domain; a preferred transmembrane domain is the CD86 transmembrane domain;
Cyto is an intracellular domain; a preferred intracellular domain is the CD86 intracellular domain.

### Vector of the Present Invention

In the present invention, the term "vector" generally refers to a nucleic acid molecule capable of transporting another nucleic acid to which it has been linked. One type of vector is a "plasmid," which refers to a circular double-stranded DNA loop into which other DNA segments can be ligated. Another type of vector is a viral vector, where other DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome, such as non-autonomously replicating naked RNA polynucleotides, naked DNA polynucleotides, polynucleotides composed of DNA and RNA on the same strand, poly-lysine-conjugated DNA or RNA, peptide-conjugated DNA or RNA, liposome-conjugated DNA, etc. In addition, certain vectors are capable of directing the expression of genes to which they are operatively linked. Such vectors are referred to herein as "recombinant expression vectors" (or simply, "expression vectors"). In general, expression vectors for use in recombinant DNA techniques are often in the form of plasmids. In the present specification, "plasmid" and "vector" are used interchangeably as the plasmid is the most commonly used form of vector.

As used herein, "the vector of the present invention" refers to a vector containing the nucleic acid construct of the present invention. Preferably, the vector of the present invention is a plasmid.

### Engineered TIL Cell of the Present Invention

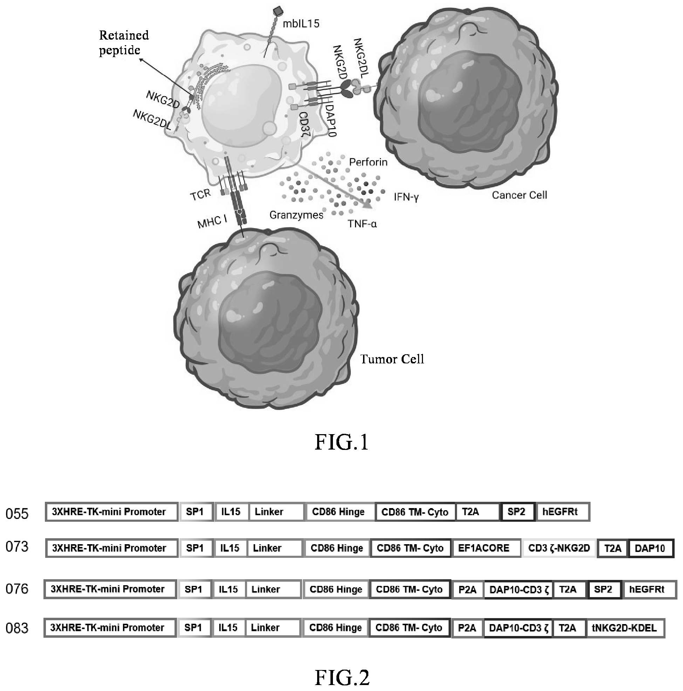

As used herein, the terms "engineered TIL cell of the present invention" or "TIL cell of the present invention" are used interchangeably and both refer to TIL cells capable of expressing the fusion protein of the present invention and/or TIL cells containing the vector of the present invention. A schematic diagram illustrating the recognition and killing of tumor cells by the TIL cells of the present invention is shown in Figure 1.

The engineered TIL cells of the present invention express the DAP10 and CD3ζ fusion protein of the present invention as an activation signal. After NKG2D on the engineered TIL cells binds to NKG2DL on the surface of tumor cells, the TIL cells can be successfully activated by the DAP10 and CD3ζ fusion protein to kill the tumor cells.

In one embodiment, the engineered TIL cells of the present invention further express the retention fusion protein of the present invention, which fuses the extracellular domain of NKG2D with the KDEL sequence. The endogenous NKG2DL transiently expressed after TIL cell activation specifically binds to the NKG2D region in the retention fusion protein, thereby being retained in the endoplasmic reticulum rather than being expressed on the TIL cell membrane, preventing mutual recognition and fratricide among TIL cells, reducing the occurrence of cell fragility, and increasing cell viability.

In one embodiment, the engineered TIL cells of the present invention further express the membrane-bound IL-15 of the present invention.

In one embodiment, the engineered TIL cells of the present invention further express the molecular switch element of the present invention. When a safety risk arises with the engineered TIL cells of the present invention, the molecular switch element can serve as a target, and corresponding drugs can be used to eliminate the cells at risk. The molecular switch element of the present invention is selected from the group consisting of: hEGFRt, BCMA, and CD20. Preferably, the molecular switch element of the present invention is hEGFRt.

### Pharmaceutical Composition of the Present Invention

The pharmaceutical composition of the present invention may comprise the fusion protein of the present invention or the immune effector cells of the present invention (e.g., the engineered TIL cells of the present invention) and one or more pharmaceutically acceptable carriers, diluents, excipients, and adjuvants. These compositions can be suitable for use in treating the therapeutic indications described herein.

The composition can be a liquid solution, suspension, emulsion, sustained-release formulation, or powder, and can be formulated with a pharmaceutically acceptable carrier. The composition can be formulated into suppositories using traditional binders and carriers such as triglycerides. A "pharmaceutically acceptable carrier" refers to a carrier matrix or vehicle that does not interfere with the effectiveness of the biological activity of the active ingredient and is not toxic to the host or subject.

The fusion protein or immune effector cells can be delivered with a pharmaceutically acceptable vehicle. In one embodiment, the vehicle can enhance stability and/or delivery properties. Vehicles such as artificial membrane vesicles (including liposomes, niosomes, nanoliposomes, etc.), microparticles or microcapsules, or colloidal formulations containing pharmaceutically acceptable polymers.

Pharmaceutical compositions containing one or more fusion proteins or immune effector cells can be formulated as sterile injectable aqueous or oleaginous suspensions according to methods known in the art and using suitable one or more dispersing or wetting agents and/or suspending agents. The sterile injectable preparation can be a sterile injectable solution or suspension in a non-toxic parenterally acceptable diluent or solvent.

In the present invention, the term "adjuvant" generally refers to any substance that assists or modulates the action of a drug, including but not limited to immunological adjuvants, which enhance or diversify the immune response to an antigen.

In the present invention, the term "subject" can be a mammal in need of treatment, such as a human or a veterinary patient (e.g., a rodent, such as a mouse or rat, cat, dog, cow, horse, sheep, goat, or other livestock). In some embodiments, a "subject" can be a clinical patient, a clinical trial volunteer, an experimental animal, etc. The subject may be suspected of having a disease characterized by cell proliferation or at risk of developing a disease characterized by cell proliferation, diagnosed with a disease characterized by cell proliferation, or confirmed as a control subject without a disease characterized by cell proliferation. As described herein, diagnostic methods for diseases characterized by cell proliferation and clinical classification of such diagnoses are known to those skilled in the art.

The pharmaceutical composition of the present invention can be used to treat tumors. In the present invention, the term "tumor" or "tumor cell" generally refers to or describes the physiological condition in mammals that is typically characterized by unregulated cell growth. Examples of tumors comprise, but are not limited to, carcinomas, lymphomas, blastomas (including medulloblastomas and retinoblastomas), sarcomas (including liposarcomas and synovial cell sarcomas), neuroendocrine tumors (including carcinoid tumors, gastrinomas, and islet cell carcinomas), mesothelioma, schwannomas (including acoustic neuromas), meningiomas, adenocarcinomas, and melanomas. "Tumor cells" can further comprise "solid tumors," which refers to tumors selected from the group consisting of: gastrointestinal cancer, pancreatic cancer, glioblastoma, cervical cancer, ovarian cancer, bladder cancer, hepatoma, breast cancer, colon cancer, rectal cancer, colorectal cancer, endometrial or uterine cancer, salivary gland cancer, kidney cancer, prostate cancer, vulvar cancer, thyroid cancer, hepatic carcinoma, anal cancer, penile cancer, testicular cancer, esophageal cancer, bile duct tumor, and head and neck cancer.

Main Advantages of the Present Invention Comprises:
(1) After NKG2D on the surface of the engineered TIL cell of the present invention recognizes NKG2DL on the surface of a tumor cell, the TIL cell can be effectively activated by the DAP10 and CD3ζ fusion protein of the present invention, thereby enabling the TIL cell to effectively kill the tumor cell.
(2) Endogenous NKG2DL expressed when the engineered TIL cell of the present invention is activated can be retained inside the cell by the retention fusion protein of the present invention, thereby avoiding fratricide between TIL cells, preventing fragility during TIL cell culture, and improving the viability of the TIL cells.
(3) The engineered TIL cells of the present invention can not only kill tumor cells by recognizing antigen peptides presented by MHC-I molecules on tumor cells via TCR, but can also kill tumors via the NKG2D-NKG2DL pathway. This expands the broad-spectrum recognition ability of traditional TIL cells against tumor cells, thereby enhancing the killing ability of TIL cells and effectively preventing immune evasion and recurrence of solid tumors.
(4) The engineered TIL cells of the present invention can also express a membrane-bound IL-15 fusion protein constructed to contain a CD86 intracellular domain, thereby significantly enhancing the stemness and killing persistence of the TIL cells.
(5) The engineered TIL cells of the present invention can induce expression of the fusion protein of the present invention via a hypoxia-responsive promoter under the characteristic hypoxic environment of solid tumors.
(6) The engineered TIL cells of the present invention can also express an immune molecular switch, such as hEGFRt, thereby avoiding potential toxicity arising from sustained expression of exogenous proteins and improving the safety of clinical treatment.

The present invention is further described below in conjunction with specific examples. It should be understood that these examples are only for illustrating the present invention and are not intended to limit the scope of the present invention. Experimental methods without specific conditions indicated in the following examples are generally performed under conventional conditions, such as those described in Sambrook et al., Molecular Cloning: A Laboratory Manual (New York: Cold Spring Harbor Laboratory Press, 1989), or according to the manufacturer's recommendations. Unless otherwise indicated, percentages and fractions are weight percentages and weight fractions.

The sequence information involved in the following examples is shown in Table A:

**Table A: Nucleotide or Amino Acid Sequences of Elements**

| **Element** | **Sequence** | **SEQ ID NO:** |
|---|---|---|
| 3×HRE-TK-mi ni promoter nucleotide sequence | GCTAACATAACCCGGGAGGTACCGAGCTCTGTCACGTCCTGCACG ACTCTAGTTGTCACGTCCTGCACGACTCTAGTTGTCACGTCCTGCA CGACGCTAGCTCGAGATCCGGCCCCGCCCAGCGTCTTGTCATTGG CGAATTCGAACACGCAGATGCAGTCGGGGCGGCGCGGTCCGAGG TCCACTTCGCATATTAAGGTGACGCGTGTGGCCTCGAACACCGAG CGACCCTGCAGCGACCCGCTTAA | 4 |
| Signal peptide SP1 amino acid sequence | MDWTWILFLVAAATRVHS | 5 |
| IL-15 amino acid sequence | NWVNVISDLKKIEDLIQSMHIDATLYTESDVHPSCKVTAMKCFLLEL QVISLESGDASIHDTVENLIILANNSLSSNGNVTESGCKECEELEEKNI KEFLQSFVHIVQMFINTS | 6 |
| LINKER amino acid sequence | SGGGSGGGGSGGGGSGGGGSGGGS | 7 |
| CD86 hinge region amino acid sequence | LEDPQPPPDHIP | 8 |
| CD86 transmembrane and intracellular region (CD86TM-Cyt o) amino acid sequence | WITAVLPTVIICVMVFCLILWKWKKKKRPRNSYKCGTNTMEREESEQ TKKREKIHIPERSDEAQRVFKSSKTSSCDKSDTCF | 9 |
| T2A peptide amino acid sequence | EGRGSLLTCGDVEENPGP | 10 |
| SP2 (GM-CSF receptor a signal peptide) amino acid sequence | MLLLVTSLLLCELPHPAFLLIP | 11 |
| EF1acore promoter nucleotide sequence | GGGCAGAGCGCACATCGCCCACAGTCCCCGAGAAGTTGGGGGGA GGGGTCGGCAATTGAACGGGTGCCTAGAGAAGGTGGCGCGGGGT AAACTGGGAAAGTGATGTCGTGTACTGGCTCCGCCTTTTTCCCGA GGGTGGGGGAGAACCGTATATAAGTGCAGTAGTCGCCGTGAACG TTCTTTTTCGCAACGGGTTTGCCGCCAGAACACAG | 12 |
| CD3ζ-NKG2D amino acid sequence | MRVKFSRSADAPAYQQGQNQLYNELNLGRREEYDVLDKRRGRDPE MGGKPQRRKNPQEGLYNELQKDKMAEAYSEIGMKGERRRGKGHDG LYQGLSTATKDTYDALHMQALPPRGWIRGRRSRHSWEMSEFHNYNL DLKKSDFSTRWQKQRCPVVKSKCRENASPFFFCCFIAVAMGIRFIIMV TIWSAVFLNSLFNQEVQIPLTESYCGPCPKNWICYKNNCYQFFDESKN WYESQASCMSQNASLLKVYSKEDQDLLKLVKSYHWMGLVHIPTNG SWQWEDGSILSPNLLTIIEMQKGDCALYASSFKGYIENCSTPNTYICM QRTV | 13 |
| DAP10 amino acid sequence | MIHLGHILFLLLLPVAAAQTTPGERSSLPAFYPGTSGSCSGCGSLSLPL LAGLVAADAVASLLIVGAVFLCARPRRSPAQDGKVYINMPGRG | 14 |
| DAP10-CD3ζ amino acid sequence | MIHLGHILFLLLLPVAAAQTTPGERSSLPAFYPGTSGSCSGCGSLSLPL LAGLVAADAVASLLIVGAVFLCARPRRSPAQDGKVYINMPGRGMRV KFSRSADAPAYQQGQNQLYNELNLGRREEYDVLDKRRGRDPEMGG KPQRRKNPQEGLYNELQKDKMAEAYSEIGMKGERRRGKGHDGLYQ GLSTATKDTYDALHMQALPPR | 1 |
| P2A peptide amino acid sequence | ATNFSLLKQAGDVEENPGP | 15 |
| hEGFRt amino acid sequence | RKVCNGIGIGEFKDSLSINATNIKHFKNCTSISGDLHILPVAFRGDSFTH TPPLDPQELDILKTVKEITGFLLIQAWPENRTDLHAFENLEIIRGRTKQ HGQFSLAVVSLNITSLGLRSLKEISDGDVIISGNKNLCYANTINWKKLF GTSGQKTKIISNRGENSCKATGQVCHALCSPEGCWGPEPRDCVSCRN VSRGRECVDKCNLLEGEPREFVENSECIQCHPECLPQAMNITCTGRGP DNCIQCAHYIDGPHCVKTCPAGVMGENNTLVWKYADAGHVCHLCH PNCTYGCTGPGLEGCPTNGPKIPSIATGMVGALLLLLVVALGIGLFM | 16 |
| tNKG2D-KDE L amino acid sequence | MALPVTALLLPLALLLHAARPLFNQEVQIPLTESYCGPCPKNWICYK NNCYQFFDESKNWYESQASCMSQNASLLKVYSKEDQDLLKLVKSY HWMGLVHIPTNGSWQWEDGSILSPNLLTIIEMQKGDCALYASSFKGY IENCSTPNTYICMQRTVKDEL | 2 |

### Example 1: Preparation of TIL Cells Containing the Target Gene

### 1. Plasmid Construction

The CDS sequences of IL-15, DAP10, NKG2D, CD3ζ, and the third and fourth extracellular domains of EGFR were obtained from NCBI, and codon optimization was performed on some sequences.

As shown in Figure 2, four constructs 055, 073, 076, and 083 were constructed respectively. The molecular sequences of each construct were synthesized via whole gene synthesis by Genescript. The synthesized gene sequences were cloned into the base vector pLV-EF1a-c-MYC-IRES-EGFP (purchased from Wuhan Miaoling Company to obtain the 055, 073, 076, and 083 plasmids.

### 2. Transformation and Plasmid Extraction

Trans109 competent cells were taken from -80°C and quickly placed on ice. After thawing, the target plasmids (055, 073, 076, 083) were added and mixed, and then incubated on ice for 25 minutes. Heat shock was performed in a 42°C water bath for 45 seconds, followed by quick transfer back to ice and incubation for 2 minutes. 700 µl of sterile LB medium without antibiotics was added, mixed, and then incubated at 37°C with 200 rpm shaking for 70 minutes for recovery. The bacterial culture was centrifuged at 5000 rpm for 1 minute to collect the cells, which were then spread on LB agar plates containing the corresponding antibiotics and placed in a 37°C incubator overnight. Single colonies were picked and inoculated into 1 ml of LB broth containing antibiotics and incubated at 37°C with 200 rpm for 2 hours. The culture was then expanded and incubated overnight. 150 ml of the overnight culture was centrifuged at 5,000 g for 10 minutes, and the supernatant was discarded as completely as possible to collect the bacterial pellet. The pellet was resuspended in 14 ml of Solution P1 until fully suspended. 14 ml of Solution P2 was added to completely lyse the cells. 14 ml of pre-chilled Solution P3 was added until a yellow flocculent precipitate appeared, followed by centrifugation for 5 minutes. The filtrate was collected, and 14 ml of plasmid DNA binding solution was added to the filtrate and mixed. After centrifugation for 2 minutes, the mixed solution was discarded. 10 ml of plasmid wash solution was added, followed by centrifugation for 2 minutes, and the waste liquid was discarded. 10 ml of plasmid DNA wash solution 2 (with anhydrous ethanol added) was added, followed by centrifugation for 2 minutes, and the waste liquid was discarded. This step was repeated. Residual ethanol was removed, and plasmid DNA was eluted. The plasmid DNA was passed through an endotoxin removal column and eluted by centrifugation for 1 minute to obtain endotoxin-free plasmid DNA. The obtained plasmid was transferred to a 1.5 ml centrifuge tube.

### 3. Lentivirus Preparation

The pLV-HRE-mIL-15 (055, 073, 076, 083) plasmids obtained in step 2, containing the mIL-15 gene, as well as the psPAX2 and pMD2.G vectors, were respectively purified via maxiprep. The plasmids were mixed at a ratio and transfected into HEK-293T cells (cultured in 100 mm dishes) at approximately 80% confluency using Lipo3000. The transfection system was prepared as follows: (1) Tube A: 500 µl Opti-MEM + 10 µg main plasmid + 5 µg pMD2.G + 5 µg psPAX2 + 40 µl P3000; (2) Tube B: 500 µl Opti-MEM + 40 µl Lipo3000. The mixture from Tube B was slowly added dropwise to Tube A, mixed, and allowed to stand at room temperature for 15-20 minutes.

The transfection system was added to the HEK-293T cells. After 4 hours of transfection, the supernatant was discarded, and 10 ml of DMEM complete medium was added to each dish. The cells were placed in a 37°C, 5% CO₂ incubator for continued culture. After 48 hours, the culture supernatant from the transfected 293T cells was collected, centrifuged at 400 g for 5 minutes, and filtered through a 0.45 µm filter. The obtained filtrate was the recombinant lentivirus stock solution. The lentivirus was concentrated using Utra-15 centrifugal filter devices by centrifugation at 5000 rpm for 50 minutes. The concentrated lentivirus was aliquoted and stored at -80°C for later use.

### 4. Lentivirus Titer Detection

HEK-293T cells were washed, trypsinized, and counted. The concentrated lentivirus stock was diluted in 10-fold serial dilutions (50×, 500×, 5000×, 50000×). 2.5 × 10⁵ HEK-293T cells were added to the corresponding centrifuge tubes, each containing 500 µl of medium-virus-cell mixture, mixed and transferred to a 24-well plate and incubated at 37°C with 5% CO₂. After 24 hours, the medium was replaced. After 48 hours, the infected HEK-293T cells were collected and subjected to hypoxia treatment, and the proportion of cells positive for transgene expression was detected by flow cytometry, and the viral titer was calculated. The lentiviral titer was calculated as follows: Viral titer = (m × 2.5 × 10⁵ × dilution factor) / volume of transfection, where m is the proportion of cells positive for transgene expression.

### 5. Preparation of TIL Cells

Tumor tissue pieces were placed in a 10 cm dish and washed with PBS. Necrotic areas and connective tissue were removed from the tumor pieces using sterile ophthalmic scissors or a surgical scalpel, and the tissue was minced into 1-3 mm³ fragments. The tumor fragments (e.g., lung cancer) were cultured in RPMI medium containing 10% human AB serum, 1% glutamine, 1% antibiotics, and 6000 IU/mL IL-2. If no obvious adherent cells were observed under microscopy, the medium was changed the following day. If lymphocytes did not gradually increase or cancer cells did not decrease, the medium was continued to be changed. If lymphocyte density significantly increased, such as formation of cell clusters, the cells were transferred to larger culture plates and placed in the 37°C, 5% CO₂ incubator. Culture was continued for up to 10 days, and then TIL cells were collected. The collected cell suspension was filtered through a 40 µm strainer to remove tumor fragments, centrifuged at 1000 rpm for 5 minutes, and resuspended in an appropriate amount of REP complete medium for later use.

### 6. Obtaining Lentivirus-Infected TIL Cells

Based on the lentiviral titer, TIL cells were infected at an MOI of 10. Lentivirus (MOI=10), Lentiboost (100×), and TIL cells (3.5 × 10⁵) were added to REP medium. Each centrifuge tube contained 200 µl of medium-virus-cell mixture, mixed and transferred to a 48-well plate and incubated at 37°C with 5% CO₂. After 24 hours of infection, cells were collected, centrifuged at 1000 rpm, and the virus-containing medium was discarded. An appropriate amount of REP (AIM V medium: A1640 medium = 1:1) complete medium was added to resuspend the cells, which were then transferred to a 24-well plate and incubated at 37°C with 5% CO₂. After 72 hours of culture, cells were collected.

1 × 10⁶ of the above cells were transferred to a new 24-well plate and placed in a hypoxia chamber (1% oxygen concentration) for hypoxia treatment (experimental group). The remaining TIL cells (as the non-hypoxia control group) were transferred to a new 24-well plate and cultured at 37°C with 5% CO₂. TIL cells infected with lentivirus containing constructs 055, 073, 076, and 083 were named 055 TIL, 073 TIL, 076 TIL, and 083 TIL, respectively.

### Example 2: Flow Cytometric Detection of Lentiviral Infection Positive Rate in TILs

Cells prepared in Example 1 were divided into two groups each: a fully negative control group and a test group, with each group containing 50 µl of cell suspension, i.e. 2.5 × 10⁵ cells). Anti-IL-15 antibody (Beijing Baixinyi Biotechnology Co., Ltd., A09D21-9E) was added to the test group and incubated at room temperature for 15 minutes. After incubation, 1 ml of PBS containing 2% FBS was added, and the cells were centrifuged at 400 g for 5 minutes. The supernatant was discarded, and 50 µl of FITC goat anti-human IgG Fcγ antibody (BioLegend, 398006) antibody mixture prepared in PBS containing 2% FBS was added to the cells in the test group and incubated at room temperature for 10 minutes. 1 ml of PBS containing 2% FBS was added, and the cells were centrifuged at 400 g for 5 minutes. The supernatant was discarded, and 50 µl of 7-AAD mixture prepared in PBS containing 2% FBS was added to the cells in the test group and incubated at room temperature for 7 minutes. 1 ml of PBS containing 2% FBS was added to both the fully negative control and test groups, and the cells were centrifuged at 400 g for 5 minutes. The supernatant was discarded, and the cells were resuspended in 200 µl of PBS containing 2% FBS for flow cytometric analysis.

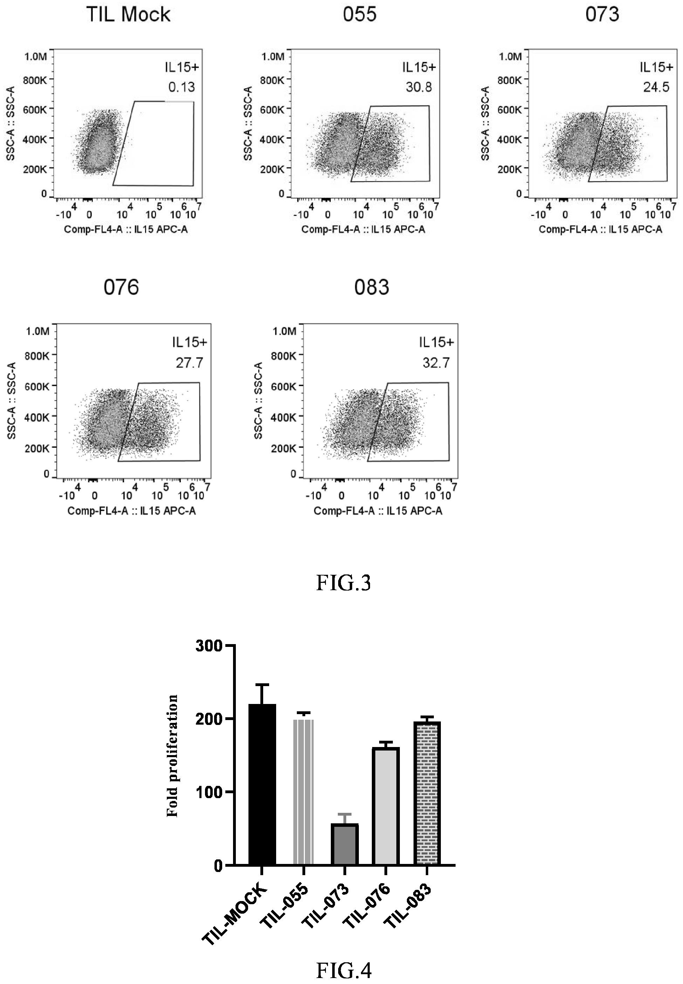

The results are shown in Figure 3. Flow cytometry data showed that the positive rates for 055, 073, 076, and 083 TILs were 30.8%, 24.5%, 27.7%, and 32.7%, respectively, all showing relatively high positive rates, with 083 TIL exhibiting an unexpectedly higher positive rate (32.7%) compared to the others.

### Example 3: Detection of Proliferation Ability and Viability of TIL Cells

TIL cells from each group were co-cultured with feeder cells (IL-21 NK cell expansion reagent, Zhongying Bio, Cat# ZY-NKZ-0104) at a ratio of 1:25. The cell density was adjusted to 5 × 10⁵ cells/ml and cultured in REP medium (RPMI 1640:AIM-V = 1:1) in 6-well plates at 2 ml/well. Cell counting was performed every two days, and fresh medium was added to adjust the cell density to 5 × 10⁵ cells/ml. Cells were continuously cultured for 14 days, and cell counts, viability, and stemness were determined.

The results of the proliferation assay are shown in Figure 4. The proliferation fold increases for control TIL, 055 TIL, 073 TIL, 076 TIL, and 083 TIL after 14 days of culture were 220, 203, 57, 167, and 198, respectively. The proliferation abilities of 055 TIL and 083 TIL were essentially the same as the control TIL, while 076 TIL showed a slight decrease in proliferation; 073 TIL exhibited a significantly reduced proliferation, far lower than those in other groups.

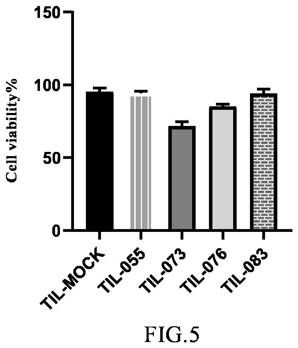

The results of cell viability testing are shown in Figure 5. Control TIL, 055 TIL, and 083 TIL showed high viabilities of 95%, 94%, and 94%, respectively; 076 TIL had slightly reduced viability at 85%; 073 TIL showed significantly reduced viability, severely affecting cell expansion, with viability only at 71%.

### Example 4: Detection of Stem-like Cell Proportion in TIL Cells

Cells from each group prepared in Example 1 were divided into two groups each: a fully negative control group and a test group, with a volume of 50 µl (2.5 × 10⁵ cells) in each group. 50 µl of Alexa Fluor^{®} 700 anti-human CD45RA antibody (BioLegend, 304120) and APC anti-human CD62L antibody (BioLegend, 304810) and antibody mixture prepared in PBS containing 2% FBS were added to the test group and incubated at room temperature for 8 minutes. 50 µl of 7-AAD mixture prepared in PBS containing 2% FBS was added to the cells in the test group and incubated at room temperature for 7 minutes. 1 ml of PBS containing 2% FBS was added to both the fully negative control and test groups, and the cells were centrifuged at 400 g for 5 minutes. The supernatant was discarded, and the cells were resuspended in 200 µl of PBS containing 2% FBS for flow cytometric analysis.

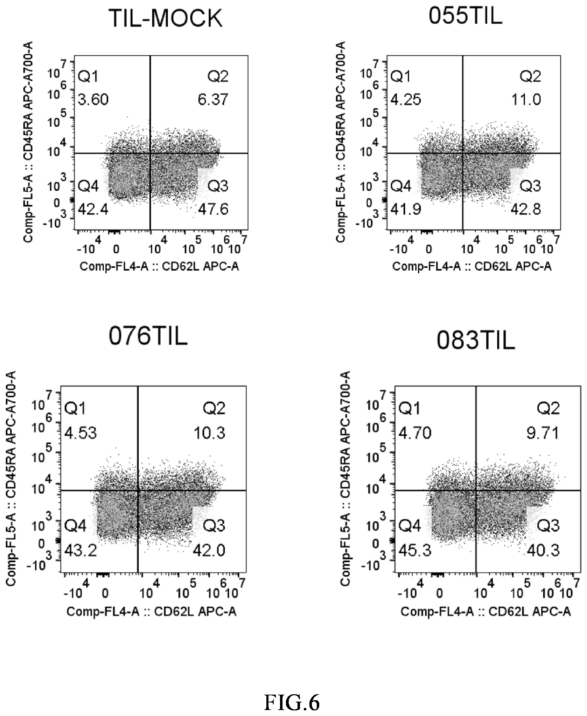

The results are shown in Figure 6. The proportion of stem-like T cells, as indicated by the percentage of CD45RA and CD62L double-positive cells, was increased from 6.37% in uninfected TIL cells (control TIL) to 11% (055 TIL), 10.3% (076 TIL), and 9.71% (083 TIL), respectively.

This indicates that the membrane-bound IL-15 construct of the present invention significantly increased the proportion of stem-like T cells in TIL cells. Furthermore, the membrane-bound DAP10-CD3ζ fusion protein had essentially no effect on the proportion of stem-like T cells.

### Example 5: Detection of IFN-γ Secretion by TIL Cells

TILs were co-cultured with CALU6, HELA, or HCC827 cells with high expression of NKG2DL at a ratio of TIL:Tumor cell = 3:1 for 18 hours, the supernatant was collected, and IFN-γ secretion was detected using an IFN-γ ELISA kit (Human IFN-γ Precoated ELISA Kit, Dakewe, 2307-3) following the manufacturer's instructions: Sample Addition: diluted cytokine standard was added to standard wells at 100 µl/well. Samples were diluted with Dilution Buffer R (1×), and added to sample wells at 100 µl/well, covered with sealing film and incubated at room temperature for 2 hours. Washing: the liquid from wells was discarded, 1× Wash Buffer working solution was added at 300 µl/well. Liquid was discarded after 1 minute. Repeated for 3 times, and blotted dry on filter paper each time. Detection Antibody Addition: Biotinylated antibody working solution was added at 100 µl/well. The plate was covered with sealing film and incubated at room temperature for 1 hour. The washing step was repeated. Enzyme Addition: Streptavidin-HRP working solution was added at 100 µl/well. The plate was covered with sealing film and incubated at room temperature (18-25°C) for 30 minutes. The washing was repeated. Color Development: TMB was added at 100 µl/well, and the plate was incubated at room temperature in the dark for 5-30 minutes. The reaction was stopped based on color intensity (dark blue). Typically, 10-20 minutes yielded good results. Stop reaction based on color intensity (dark blue). Typically, 10-20 minutes yields good results. Reaction Termination: Stop Solution was quickly added at 100 µl/well to stop the reaction. The OD was read at 450 nm using a microplate reader. The data were analyzed using the four-parameter method.

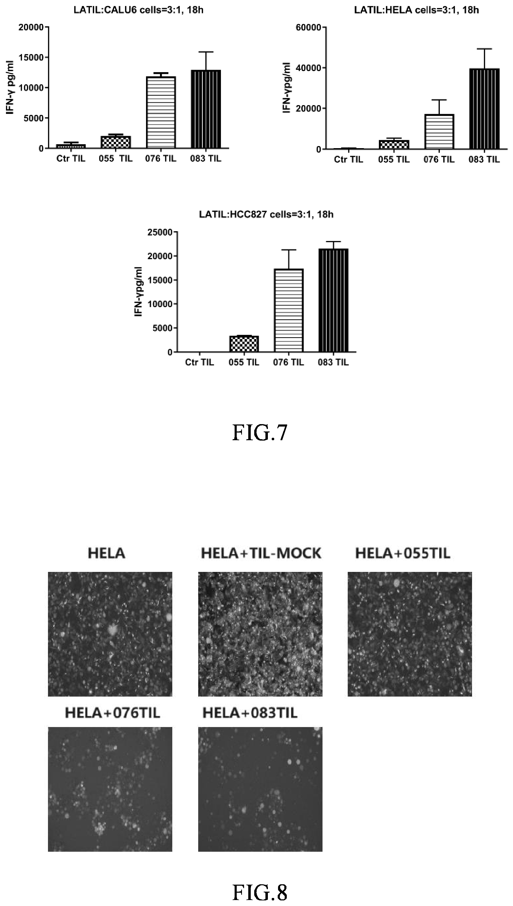

The results are shown in Figure 7 and Tables 1-3. 076 TIL and 083 TIL co-cultured with various tumor cells secreted significantly higher amounts of IFN-γ compared to uninfected control TIL and 055 TIL.

**Table 1: IFN-γ Secretion (pg/ml) by TILs Co-cultured with CALU6 Cells in Each Group**

| IFN-γ Secretion (pg/ml) | Control TIL | 055 TIL | 076 TIL | 083 TIL |
|---|---|---|---|---|
| Parallel 1 | 362.79 | 2300.22 | 12374.58 | 15885.41 |
| Parallel 2 | 981.58 | 1785.62 | 11264.54 | 9975.12 |
| Parallel 3 | 500.87 | 2000.44 | 11345.56 | 10345.23 |

**Table 2: IFN-γ Secretion (pg/ml) by TILs Co-cultured with HELA Cells in Each Group**

| IFN-γ Secretion | Control TIL | 055 TIL | 076 TIL | 083 TIL |
|---|---|---|---|---|
| Parallel 1 | 319.63 | 5546.15 | 25179.85 | 62579.38 |
| Parallel 2 | 799.08 | 3546.15 | 10546.15 | 40090.04 |
| Parallel 3 | 600.96 | 4445.56 | 14506.78 | 35070.56 |

**Table 3: IFN-γ Secretion (pg/ml) by TILs Co-cultured with HCC827 Cells in Each Group**

| IFN-γ Secretion | Control TIL | 055 TIL | 076 TIL | 083 TIL |
|---|---|---|---|---|
| Parallel 1 | 394.83 | 3800.07 | 25300.07 | 27094.69 |
| Parallel 2 | 282.54 | 3420.59 | 13402.73 | 23023.84 |
| Parallel 3 | 273.23 | 3500.69 | 14030.49 | 21560.89 |

These results indicate that compared to 055 TIL, which relies solely on TCR for tumor cell recognition, 076 TIL and 083 TIL expressing the membrane-bound DAP10-CD3ζ fusion protein of the present invention can more efficiently utilize the NKG2D-NKG2DL pathway to activate TIL cells in addition to TCR recognition. Furthermore, 083 incorporating the endoplasmic reticulum retention technology to prevent TIL fragility, exhibited higher viability than 076 TIL; this higher viability makes them more readily activated during co-culture with target cells, thus demonstrating higher IFN-γ secretion.

### Example 6: In Vitro Cytotoxicity Assays of TILs

1 × 10⁵ tumor cells (Calu-6 (ATCC, HTB-56^{™}), HELA (ATCC, CRM-CCL-2^{™}), HCC827 (CRL-2868), or A-375 B2M KO (Guangzhou Yuanjing, Cat# YKO-H486)) were seeded in 24-well plates. After 24 hours, TIL cells were diluted to a density of 1.5 × 10⁶/ml. The culture medium in the 24-well plates was aspirated. 1 ml of TIL cell suspension was added to each well. After 24 hours, the culture medium was aspirated and centrifuged, and the resulting supernatant was used for ELISA. The cells in the wells were washed with PBS, and tumor cell viability was assessed using the CCK-8 method.

Microscopic observation and photographing were performed after TILs from each group co-cultured with tumor cells (HELA) at a ratio of 3:1 for 24 hours. The results are shown in Figure 8: In the HELA only group and the HELA + control TIL group, HELA cells grew well, forming a dense monolayer. In the HELA + 055 TIL group, HELA cell growth was slower, failing to reach confluence, with some cells becoming rounded and detached. In the HELA + 076 TIL and HELA + 083 TIL groups, a large area of HELA cells became rounded and detached.

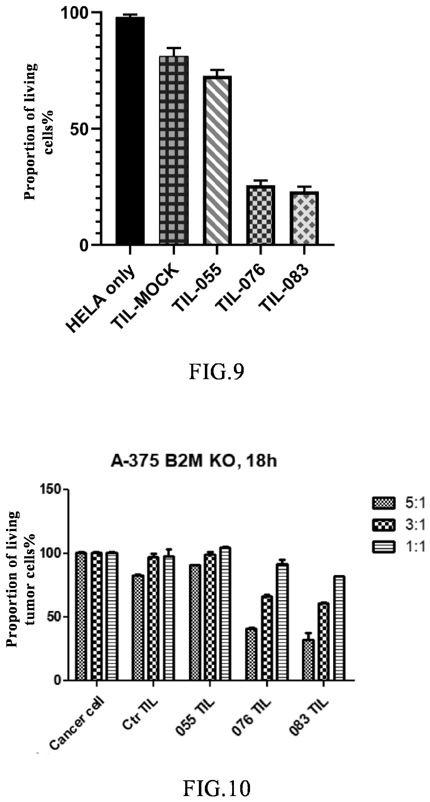

Cytotoxicity of TILs against HELA tumor cells at an E:T ratio of 3:1 is shown in Figure 9 and Table 4. Compared with control TIL and 055 TIL, 076 TIL and 083 TIL exhibited significantly enhanced killing ability against tumor cells, approximately 3-fold higher.

**Table 4: Proportion of Living Target Cells after Killing of HELA Cells by Different TILs (%)**

| Proportion of living cells | HELA only | TIL-MOCK | 055 TIL | 076 TIL | 083 TIL |
|---|---|---|---|---|---|
| Parallel 1 | 98.2 | 85.4 | 73.8 | 25.6 | 23.6 |
| Parallel 2 | 99.3 | 80.9 | 75.5 | 28.4 | 25.7 |
| Parallel 3 | 97.0 | 78.5 | 70.3 | 24.3 | 21.3 |

Cytotoxicity of TILs against A-375 B2M KO tumor cells at E:T ratios of 1:1, 3:1, and 5:1 is shown in Figure 10 and Table 5. Modified 076 and 083 TILs showed significantly enhanced killing effects against the melanoma cell line A375 (B2M KO) compared with 055 TIL and unmodified control TIL, in a dose-dependent manner. At higher effector-to-target ratios, the cytotoxicity was more pronounced, reaching approximately 60% (100% minus the proportion of living cells).

**Table 5: Proportion of Living Target Cells after Killing of A-375 B2M KO by Different TILs (%)**

| E:T Ratio | 5:1 | | 3:1 | | 1:1 | |
|---|---|---|---|---|---|---|
| A375 B2MKO only | 98.6 | 99.7 | 97.8 | 99.4 | 98.4 | 98.7 |
| Ctr TIL+A-375 B2MKO | 82.7 | 83.7 | 99.2 | 94.4 | 91.9 | 99.5 |
| 055 TIL+A-375 B2MKO | 91.1 | 91.7 | 98.7 | 97.5 | 99.4 | 99.2 |
| 076 TIL+A-375 B2MKO | 41.8 | 40.7 | 65.6 | 68.3 | 95.6 | 87.7 |
| 083 TIL+A-375 B2MKO | 26.7 | 37.7 | 61.6 | 61.2 | 81.6 | 82.5 |

### Example 7: Antibody-Dependent Cell-Mediated Cytotoxicity (ADCC) Assay

Antibody dependent cell-mediated cytotoxicity (ADCC) refers to the process where the Fab segment of an antibody binds to an antigenic epitope on a target cell, and its Fc segment binds to Fc receptors on effector cells (NK cells, macrophages, neutrophils, etc.), mediating the direct killing of target cells by effector cells. Cetuximab binds to EGFR and mediates target cell death by ADCC. Rituximab does not bind to EGFR and does not cause ADCC-mediated cell death, so it can serve as a control to demonstrate specificity.

NK cells were isolated and expanded from peripheral blood of healthy volunteers using an NK cell culture kit (purchased from Fujian Sanyi Hematopoietic Technology Co., Ltd., CT-001) according to the manufacturer's instructions and labeled with CytoTell Blue. Target cells (076 LV-infected TILs) were resuspended in medium at a density of 5 × 10⁵ cells/ml, and 1 ml was added to each of three wells in a 6-well plate. Cetuximab (Merck KGaA, Germany) or Rituximab (Roche Diagnostics GmbH, H0334) was added to the wells to a final concentration of 200 µg/ml, and an equal volume of PBS was added to the control well, mixed and incubated at room temperature for 40 minutes. NK cells were resuspended in medium at a density of 5 × 10⁶/ml, and 1 ml was added to each of the three wells in the 6-well plate, achieving an effector-to-target ratio of 10:1. The final concentrations of cetuximab and rituximab in the system were 100 µg/ml, and the total volume was 2 mL. The system was mixed and incubated in a 37°C incubator for 24 hours. After 24 hours, changes in apoptosis levels were detected (PE Annexin-V Apoptosis Detection Kit, BD Biosciences, 559763).

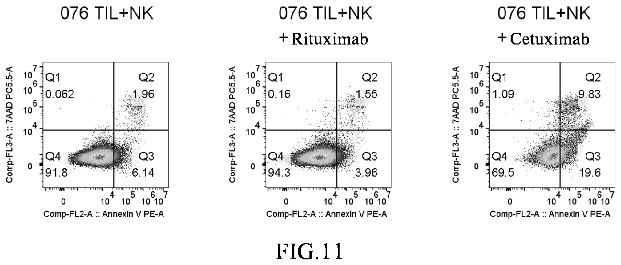

The results are shown in Figure 11. The apoptosis rate of 076 TILs was between 5.5-8.1% when incubated with NK cells or with NK cells plus rituximab (which recognizes CD20), remaining at a relatively low level. In the presence of both NK cells and cetuximab (which recognizes EGFR), the apoptosis rate of 076 TILs was approximately 30%. These experimental results indicate that hRGFRt can function as a specific molecular switch, enhancing the safety of clinical use.

### Discussion

The present inventors have for the first time provided structurally novel and unique membrane-bound DAP10 and CD3ζ fusion proteins. The membrane-bound DAP10-CD3ζ fusion protein of the present invention can unexpectedly and efficiently initiate the activation of TIL cells, leading to more effective killing of tumor cells.

In the present invention, after the NKG2D on the engineered TIL cell surface recognizes NKG2DL on the tumor cell surface, the TIL cell can be further effectively activated via the fusion protein of the present invention, thereby enabling the TIL cell to effectively kill the tumor cell.

Taking 083 TIL as an example, it utilizes a hypoxia-inducible promoter to regulate the expression of the DAP10 and CD3ζ fusion protein of the present invention. Only when the TIL cells reach the tumor tissue or its surrounding environment do they express the membrane-bound DAP10-CD3ζ fusion protein. After recognizing the tumor cell via NKG2D-NKG2DL, the TIL cell can be activated via the DAP10 and CD3ζ functional elements to effectively kill the tumor cell.

The engineered TIL cells of the present invention can not only kill tumor cells by recognizing antigen peptides presented by MHC-I molecules on tumor cells via TCR, but can also kill tumors via the NKG2D-NKG2DL pathway, effectively preventing immune evasion and recurrence of solid tumors.

Furthermore, the present invention provides a structurally novel and unique retention fusion protein. Experiments have shown that the retention fusion protein of the present invention can be effectively retained in the endoplasmic reticulum, thereby retaining endogenously expressed NKG2DL inside the TIL cells, avoiding fratricide between TIL cells, preventing fragility during the TIL cell culture process, and thus significantly improving the viability of TIL cells.

Additionally, the engineered TIL cells of the present invention can further express a structurally novel and unique membrane-bound IL-15, thereby further enhancing the stemness of TIL cells.

Moreover, the engineered TIL cells of the present invention can further express an hEGFRt molecular switch (e.g., 076 TIL). Via ADCC, they can specifically induce apoptosis of the relevant TILs, ensuring the safety of TIL therapy for clinical use. At the same time, cetuximab can be used for flow cytometry detection as a positive rate indicator.

All documents mentioned in the present invention are cited as references in this application, as if each document was individually cited as a reference. Furthermore, it should be understood that after reading the above teaching content of the present invention, those skilled in the art can make various changes or modifications to the present invention, and these equivalent forms also fall within the scope defined by the appended claims of this application.

## Claims

1. An engineered TIL cell with enhanced ability to recognize and kill tumors, wherein the engineered TIL cell expresses an exogenous fusion protein selected from the group consisting of: an activation fusion protein, a retention fusion protein, and a combination thereof;
wherein the activation fusion protein is a membrane-bound DAP10-CD3ζ fusion protein;
and wherein the retention fusion protein comprises: an NKG2D extracellular domain and a KDEL sequence.

2. The engineered TIL cell of claim 1, wherein the activation fusion protein comprises:
(1) an extracellular domain, wherein the extracellular domain comprises a DAP10 element or an active fragment thereof;
(2) a transmembrane domain; and
(3) an intracellular signaling domain, wherein the intracellular signaling domain comprises a DAP10 intracellular domain and CD3ζ;
wherein the extracellular domain and the intracellular signaling domain are linked in tandem via the transmembrane domain.

3. The engineered TIL cell of claim 2, wherein the intracellular signaling domain further comprises a co-stimulatory domain.

4. The engineered TIL cell of claim 1, wherein the amino acid sequence of the activation fusion protein is set forth in SEQ ID NO: 1.

5. The engineered TIL cell of claim 1, wherein the amino acid sequence of the retention fusion protein is set forth in SEQ ID NO: 2.

6. The engineered TIL cell of claim 1, wherein the engineered TIL cell has the activation fusion protein on its cell membrane; and/or the engineered TIL cell has the retention fusion protein in its endoplasmic reticulum or Golgi apparatus.

7. The engineered TIL cell of claim 1, wherein the engineered TIL cell comprises a polynucleotide encoding the activation fusion protein and/or the retention fusion protein.

8. The engineered TIL cell of claim 1, wherein the engineered TIL cell comprises a nucleic acid construct, wherein the nucleic acid construct comprises a first expression cassette comprising a polynucleotide encoding the activation fusion protein and/or a polynucleotide encoding the retention fusion protein.

9. The engineered TIL cell of claim 8, wherein the first expression cassette further comprises a promoter selected from the group consisting of: a constitutive promoter, an inducible promoter, and a combination thereof.

10. The engineered TIL cell of claim 1, wherein the engineered TIL cell comprises a vector comprising: a polynucleotide encoding the activation fusion protein and/or a polynucleotide encoding the retention fusion protein.

11. The engineered TIL cell of claim 1, wherein the engineered TIL cell further expresses a membrane-bound IL-15 fusion protein comprising the following elements:
(i) interleukin 15 or a functionally active fragment thereof;
(ii) a transmembrane domain; and
(iii) an intracellular domain;
wherein the intracellular domain is a CD86 intracellular domain.

12. A composition, wherein the composition comprises the engineered TIL cell of claim 1 and a pharmaceutically acceptable carrier.

13. A kit, wherein the kit comprises the engineered TIL cell of claim 1, or a reagent for preparing the engineered TIL cell of claim 1, wherein the reagent is selected from the group consisting of:
(Y1) a polynucleotide encoding the activation fusion protein and the retention fusion protein; or
(Y2) a vector comprising: a polynucleotide encoding the activation fusion protein and/or a polynucleotide encoding the retention fusion protein.

14. Use of the engineered TIL cell of claim 1, or the kit of claim 13 in the manufacture of a medicament for treating a tumor.

15. A method of treating a disease, wherein the method comprises administering to a subject in need thereof the cell of claim 1 and/or the composition of claim 12.
